(19) Europäisches Patentamt
European Patent Office
Office européen des brevets

(11) EP 0 861 340 B1

(12) EUROPEAN PATENT SPECIFICATION

(45) Date of publication and mention of the grant of the patent:
**25.04.2001 Bulletin 2001/17**

(21) Application number: **96917426.7**

(22) Date of filing: **24.05.1996**

(51) Int Cl.[7]: **D04H 1/04**, D04H 1/10, A61L 15/00

(86) International application number:
**PCT/EP96/02249**

(87) International publication number:
**WO 96/37650 (28.11.1996 Gazette 1996/52)**

(54) **PROCESS FOR BINDING FIBROUS WEB**

VERFAHREN ZUR BINDEN EINER FASERSTOFFBAHN

PROCEDE DE LIAISON DE TISSU FIBREUX

(84) Designated Contracting States:
**AT BE DE DK ES FI FR GB GR IE SE**

(30) Priority: **25.05.1995 GB 9510606**

(43) Date of publication of application:
**02.09.1998 Bulletin 1998/36**

(73) Proprietor: **CAMELOT SUPERABSORBENTS LTD**
**Calgary, Alberta T2E 7L6 (CA)**

(72) Inventors:
- **DINGLEY, Roger**
  **Maidenhead, Berkshire SL6 1YP (GB)**
- **DAN, Ervin**
  **Calgary, Alberta T2R 1H3 (CA)**
- **BOUMAN, Dirk, Camelot Superabsorbents B.V.**
  **NL-7500 AM Enschede (NL)**
- **SHALTRY, Michael**
  **Charlotte, NC 28210 (US)**

(74) Representative: **Ritter, Stephen David**
**Mathys & Squire**
**100 Grays Inn Road**
**London WC1X 8AL (GB)**

(56) References cited:

| | |
|---|---|
| **EP-A- 0 188 091** | **EP-A- 0 189 102** |
| **EP-A- 0 198 683** | **EP-A- 0 361 842** |
| **EP-A- 0 671 496** | **WO-A-93/20272** |
| **US-A- 3 976 734** | **US-A- 4 186 165** |
| **US-A- 4 443 492** | **US-A- 5 382 609** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a process for binding a fibrous web which includes water-absorbent material such as water-absorbent fibers. In particular the present invention relates to binding a fibrous web containing so-called superabsorbent fibers. The invention also relates to an article of manufacture comprising a fibrous web made in accordance with the process.

Fibrous webs containing water-absorbent compositions are widely used in the manufacture of products which require high absorption capability; for example, surgical and dental sponges, tampons, sanitary napkins and pads, pant liners, adult incontinence pads, coverstock for feminine hygiene products, bandages, disposable diapers, patient underpads, mortuary pads, meat trays, wipes, domestic wipes, industrial wipes, packaging, filters, medical tray pads, fenestration drapes, other medical and surgical related articles, cable wrap, food preservation articles, seed germination pads, roofing materials, automotive trim, furniture, gasket sealants, pond liners, bedding, clothing, cement and household pet litter. Water-absorbing compositions are also used in the modification of soil to improve water-retention and increase air capacity and for a host of other applications. It will be understood that some of the referred to articles are not per se absorbent but may be rendered absorbent by means of the presence in their structure, or at their surface, of absorbent materials.

Water-absorbent compositions suitable for these and other uses may be in any suitable form including powders, fibers and filaments, with fibers being particularly preferred.

As used herein, the term "water" when used in the phrases "water-absorbing", "water-absorbent" and "water-swellable" is understood to mean not only water but also aqueous media such as, in particular electrolyte solutions such as body fluids.

The fibrous webs of the type with which the present invention is concerned are generally formed from non-water-absorbent fibers. By "non-water-absorbent" we mean that the fibers do not absorb water to an appreciable extent. Suitable materials from which these fibers may be formed include natural materials such as wood pulp or cellulose or synthetic materials such as synthetic cellulose, viscose, polyester, non-water-absorbent polymers of propylene, polyamide, and ethylene-propylene copolymer fibers, and mixtures thereof, with polyester, polyethylene and polypropylene fibers being particularly preferred. Further particularly preferred fibers are those selected from the group consisting of rayon fibers, cellulose ester fibers, protein fibers, polyamide fibers, polyester fibers, polyvinyl fibers, polyolefin fibers, polyurethane fibers, aramid fibers, glass fibers and mixtures thereof. In one alternative, the non-water-absorbent fibers may be, or may include, fibers having hollow cores such as the polyester, typically polyethylene terephthalate, fibers commercially available from E.I. DuPont de Nemours under the trade mark HOLLOWFILL.

The fibrous webs additionally include water-absorbent compositions, which are preferably water-absorbent fibers.

A number of absorbent compositions have been developed which exhibit the capacity to be water-absorbing. For example, U.S. Patent numbers 3,954,721 and 3,983,095 disclose preparations for derivatives of copolymers of maleic anhydride with at least one vinyl monomer in fibrous form. The fibrous copolymers are rendered hydrophillic and water-swellable by reaction with ammonia or an alkali metal hydroxide. U.S. Patent No. 3,810,468 discloses lightly cross-linked olefin-maleic anhydride copolymers prepared as substantially linear copolymers and then reacted with a diol or a diamine to introduce cross-linking. The resultant lightly cross-linked copolymers are treated with ammonia or an aqueous or alcohol solution of an alkali metal hydroxide. U.S. Patent No. 3,980,663 describes water-swellable absorbent articles made from carboxylic polyelectrolytes via cross-linking with glycerine diglycidyl ether.

European Published Application No. 0 268 498 describes a water-absorbent composition formed by causing a substantially linear polymer of water-soluble ethylenically unsaturated monomer blends comprising carboxylic and hydroxylic monomers to cross-link internally.

Further examples of water-absorbent compositions are those produced from a copolymer of an $\alpha,\beta$ unsaturated monomer having at least one pendant unit selected from a carboxylic acid group and derivatives thereof and a copolymerisable monomer. A proportion of the pendant units are present in the final copolymer as the free acid and a proportion as the salt of the acid. These copolymers are capable of being cross-linked, either internally or with a variety of cross-linking agents, to form the water-swellable composition. Examples of water-swellable compositions of this type can be found in U.S. Patent Nos 4,616,063, 4,705,773, 4,731,067, 4,743,244, 4,788,237, 4,813,945, 4,880,868 and 4,892,533 and European Patent Nos 0 272 074 and 0 264 208 and European Published Application No. 0 436 514.

Derivatives of carboxylic acid groups include carboxylic acid salt groups, carboxylic acid amide groups, carboxylic acid imide groups, carboxylic acid anhydride groups and carboxylic acid ester groups.

Other examples of water-absorbent compositions can be found in US 4798861, WO93/17066, WO93/2555735, WO93/24684, WO93/12275, European Published Application Nos 0 401 044, 0 269 393, 0 326 382, 0 227 305, 0 101 253, 0 213 799, 0232 121, 0 342 919, 0 233 014, 0 268 498 and 0 397 410, British Patent Application Nos 2 082 614, 2 022 505, 2 270 030, 2 269 602 and 2 126 591, U.S. Patent Nos 4,418,163, 4,418,163 3,989,586, 4,332,917, 4,338,417, 4,420,588 and 4,155,957 and French Patent Application No. 2 525 121.

Where the water-absorbent composition is in fibrous form it may be formed into a non-woven web by conventional means. Suitable means include air-laying, wet-laying and carding. In one alternative embodiment the web may be a woven web. Whether the web is woven or non-woven, the fibers may be bound, for example by the action of an adhesive and/or heat.

In WO92/02199 it is suggested that a web of non-woven fibrous water-absorbent materials could be formed without the action of an adhesive and/or heat by forming the web in the presence of water vapour. It is stated that the presence of the water vapour causes the constituents of the web including the absorbent fibers to be lightly adhered together to maintain sufficient integrity of the web including the absorbent fibers during normal use but allows the disintegration and dispersion of the fibers when the article formed from the web is placed in contact with a large volume of water, such as when the article is disposed of in the toilet. The use of water to bind the constituents of the web together is described in WO92/02199 as hydroentanglement.

This process of forming the non-woven web in the presence of water vapour has certain disadvantages and drawbacks. Where the fibers are to be air laid in the conventional manner to form the non-woven web, they are blown into a chamber and allowed to fall onto a support. If this chamber has a humid atmosphere due to the presence of water-vapour, the fibers will swell and become sticky. This is disadvantageous as the fibers will tend to congregate within the chamber, thereby disrupting the free-fall and laying of the fibers. In addition, the sticky fibers will adhere to the walls of the chamber and to other apparatus that may be associated with the air-laying process.

We have now discovered that the aforementioned drawbacks and disadvantages may be reduced or overcome if the water is applied to the fibers after the non-woven web has been formed. We have also found that water may be used as a binder where the web is woven.

Thus according to a first aspect of the present invention there is provided a process for binding a fibrous web which includes water-absorbent material wherein a pre-formed loose web is contacted with water.

By "loose web" we mean the web as formed prior binding with a binding agent. It will be understood that the term "water" as used herein includes water vapour.

The water-absorbent material is preferably in fibrous form. The fibrous web may be formed wholly from water-absorbent fibers. Where the web is formed wholly from water-absorbent fibers, water-absorbent material in particulate form may be present within the web.

Without wishing to be bound by any theory it is believed that the water causes the water-absorbent material to swell and thereby become sticky such that it adheres to the non-water-absorbent fibers, or where the fibrous web is formed wholly from water-absorbent material, particularly fibers, to each other.

The water as binder may be applied to the loose web by any suitable means. In one arrangement, the web may be immersed in water; excess water can then be removed by, for example, applying pressure to the web. In one alternative arrangement, the water is sprayed onto the web. The water may be sprayed onto one or both sides of the web. Spraying is the generally preferred means of applying the water onto the loose web. It is particularly preferred in applications where the retention of the bulk or loft of the web is important. The water may be sprayed onto the fibrous web to obtain spot welds at the points where fibers are in mutual contact. In a second alternative arrangement, the web is passed through an area containing water-vapour.

In a particularly preferred embodiment, the fibers are blown into a chamber and allowed to fall in the conventional manner onto a conveyor, the resultant web is then removed from the chamber on the conveyor and water is sprayed onto the web such that the fibers swell, become sticky and thereby bind. In one alternative embodiment, the conveyor transports the web from the chamber where air laying has taken place to a second chamber which has a humid atmosphere.

Where the percentage of water-absorbent material present in the web is low additional binders may be used. Suitable binders include cationic starch, polyvinyl alcohol, pearl starch, natural gums, such as tragacanth, karaya, and quar, vinyl acetate-acrylic acid copolymers, polyvinyl chlorides, polyvinyl acetates, ethylene-vinyl acetates, styrenebutadiene carboxylates, natural latex, synthetic latex, including polyacrylates such as polyethylacrylate and copolymers thereof, polyacrylonitriles and thermosetting cationic resins such as urea formaldehyde resins and polyamide-epichlorohydrin resins, with natural or synthetic latex being preferred. Examples of commercially available compositions which are suitable binders include AIRBOND, AIRFLEX and VINEX of Air Products Inc., HYCAR and GEON of Goodrich Chemical Company and FLUTEX of HB Fuller Company. The binder may be an integral part of the non-water absorbent fiber. One example of this is a bicomponent fiber such as fiber consisting of a polyethylene sheath around a polypropylene core fiber. As polyethylene has a lower melting point than polypropylene, the resultant web may be heated such that the polyethylene sheath melts leaving the polypropylene fibers in place. As the web is cooled the polyethylene will solidify and thereby bind the polypropylene fibers.

The binder may be applied to the web by any suitable means including dipping, and spraying including spot welding. The web having been treated by binder is allowed to cure. Curing is preferably achieved by passing the web that has been treated with the aqueous solution of the binder through an oven. During curing, cross-linking of the binder will occur and binding will thereby be effected. In order to effect cross-linking the binder will contain an amount of a

suitable cross-linking agent. One suitable cross-linking agent is N-methylol acrylamide.

The binder may include suitable additives such as defoamers, surfactants, external crosslinkers, thickeners, flame retardants, catalysts, pH adjusters, dyes and pigments, fillers, optical brighteners and sewing aids.

Where an additional binder is used, the binder may be applied to the web and cured before the water is applied. The water will then serve to bind the water-absorbent material to the fibers of the non-woven web. In one alternative embodiment the water and the binder may be applied simultaneously. In this case the binder may be applied to the web as an aqueous solution. As the web is dried the binder will cure. However, sufficient water will be retained by the water-absorbent fibers to effect the binding that is the subject of the present invention.

The use of a binder and water will be particularly advantageous where the web is a "through air" thermal bound product or the like. In general such products are of very low density, for example of the order of 0.01 g/$cm^3$, and water-absorbent material may be lost. Water-absorbent material may also be lost from other forms of webs including woven webs. Where water is added in accordance with the present invention the water will cause the water-absorbent material to be bound to the fibers of the web. The water is preferably applied before the through air system or the calendar section of a calendar bonded carded material of the forming section.

Thus according to a second aspect of the present invention there is provided a process of anchoring water-absorbent material in a fibrous web wherein a web is formed from non-water-absorbent fibers and water-absorbent material and water is applied to bind the water-absorbent material to the non-water-absorbent fibers.

The water-absorbent material is preferably water-absorbent fibers.

The fibrous web is preferably water-absorbent paper. One benefit of utilizing the process of the second aspect of the present invention is that more water-absorbent material may be held in the fibrous web and the water-absorbency of the paper will be improved.

Water may also be used to improve the properties of a non-woven web formed using conventional laying and bonding methods. Because of the nature of conventional non-woven webs that incorporate water-absorbent material, particularly water-absorbent fibers, which have been formed by conventional means, the product may disperse dust, and/or loose fibers into the environment. This can make the product difficult to store and handle.

We have discovered that if the pre-formed and pre-bound non-woven web containing water-absorbent material are contacted with sufficient water that they swell and become sticky, any dust particles or loose fibers become adhered to the fibers of the web thereby obviating the problems associated with known non-woven webs.

Thus according to a further aspect of the present invention there is provided a process for binding loose particles to a non-woven fibrous web containing water-absorbent material in which water is applied to the fibrous web.

The water-absorbent material is preferably water-absorbent fibers.

In each of the above-mentioned aspects of the present invention the water-absorbent material, which may be in the form of fibers, may be any water-absorbent material. However, the water-absorbent material is preferably a copolymer containing from about 25 to about 75 mole percent recurring units of an α,β unsaturated monomer and from about 75 to about 25 mole percent recurring units of a copolymerisable monomer. The copolymer preferably contains from about 35 to about 65 mole percent recurring units of at least one α,β unsaturated monomer and from about 65 to about 35 mole percent of at least one copolymerisable co-monomer. Most preferably, the copolymer will be an equimolar copolymer.

Suitable α,β unsaturated monomers are those bearing at least one pendant carboxylic acid unit or derivative of a carboxylic acid unit. Derivatives of carboxylic acid units include carboxylic acid salt groups, carboxylic acid amide groups, carboxylic acid imide groups, carboxylic acid anhydride groups and carboxylic acid ester groups.

Examples of suitable α,β unsaturated monomers include maleic acid, crotonic acid, fumaric acid, mesaconic acid, the sodium salt of maleic acid, the sodium salt of methyl 2-butene dicarboxylic acid, the sodium salt of itaconic acid, maleamic acid, maleamide, N-phenyl maleimide, maleimide, maleic anhydride, fumaric anhydride, itaconic anhydride, citraconic anhydride, ethyl maleic anhydride, diethylmaleate, methylmaleate and the like and mixtures thereof.

Any suitable co-monomer can be employed, Examples of suitable co-monomers include ethylene, propylene, isobutylene, $C_1$ to $C_4$ alkyl methacrylates, vinyl acetates, methyl vinyl acetates, methyl vinyl ether, isobutyl vinyl ether and styrenic compounds having the formula:

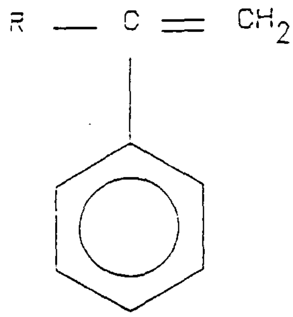

wherein R represents hydrogen or an alkyl group having 1 to 6 carbon atoms, and wherein the benzene ring is substituted or unsubstituted.

Suitable alkyl acrylates include methyl acrylate , ethyl acrylate, isopropyl acrylate, n-propyl acrylate, and the like and mixtures thereof.

Suitable alkyl methacrylates include methyl methacrylate, ethyl methacrylate, isopropyl methacrylate, n-propylmethacrylate, n-butyl methacrylate, and the like and mixtures thereof.

One alternative uncured polymer is formed from a water-soluble blend of monoethylenically unsaturated monomers. The polymer may include a comonomer. The polymer preferably comprises 50 to 95% by weight of an ethylenically unsaturated monomer and 5 to 50% by weight of a copolymerisable ethylenically unsaturated monomer.

Preferred carboxylic monomers include methacrylic acid, acrylic acid, maleic acid or anhydride, itaconic acid and ethylenically unsaturated carboxylic acids or anhydrides. Some of the carboxylic monomer units in the copolymer may be replaced by monomer units derived from an ethylenically unsaturated sulphonic acid such as 2-acrylamido-2-methylpropane sulphonic acid or allyl sulphonic acid.

The or each copolymerisable ethylenically unsaturated monomer may be selected from a water-soluble ethylenically unsaturated monomer such as acrylamide or a water-insoluble monomer, for example an olefin, such as isobutylene, an aromatic ethylenically unsaturated monomer, such as styrene or a substituted styrene, an alkyl ester of acrylic or methacrylic acid, such as methyl or ethyl acrylate or methacrylate, butyl acrylate or methacrylate or 2-ethylhexyl acrylate or methacrylate, vinyl acetate or acrylonitrile. Other monomers that may be used include ethylenically unsaturated monomers that carry a pendent group of the formula $-A_mB_nA_pR$ where B is ethyleneoxy, n is a integer of at least 2, A is propyleneoxy or butyleneoxy, m and p are each an integer less than n and preferably below 2 and most preferably zero, and R is a hydrophobic group containing at least 8 carbon atoms as described in more detail in EP-A-213799. The comonomers(s) are generally present in amounts of at least 5% and preferably at least 10% by weight based on the monomers used for forming the copolymer, and they may be present in amounts up to about 50%, generally below 45%, by weight.

The copolymer will be crosslinked. Crosslinking may be internal cross-linking or, alternatively, the binder solution may contain some external cross-linking agents. Suitable cross-linking agents include: monomers containing at least two hydroxyl groups such as alkylene glycols containing 2-10 carbon atoms and their ethers, cycloalkylene glycols, Bisphenol A, hydroxy alkylene derivatives of Bisphenol A, hydroquinone, phloroglucinol, hydroxy alkylene derivatives of diphenols, glycerol, erythritol, pentaerythritol, and mono-, di, or oligo-saccharides; heterocyclic carbonates; and monomers containing at least one amine group and at least one hydroxyl group such as ethanolamine, tris (hydroxymethyl) aminomethane, 3-amino-1-propanol, DL-1-amino-2-propanol, 2-amino-1-butanol, N,N-dimethylethanolamine, diisopropanol-amine methyl diethanol amine, triethanol amine, 2-(methylamino)ethanol and the like.

The fibrous web produced in accordance with any of the above-mentioned aspects of the invention is suitable for use in the production of articles of manufacture. Thus, in accordance with a further aspect of the present invention, there is provided an article of manufacture comprising a fibrous web made in accordance with the process of the above first, second or third aspect.

The article of manufacture is preferably selected from the group consisting of: disposable diapers; sanitary napkins; tampons; pant liners; adult incontinence pads; coverstock for feminine hygiene products; surgical and dental sponges; bandages; patient underpads; wipes; domestic wipes; industrial wipes; packaging; medical tray pads; fenestration drapes; filters; spill control materials; waste management materials; protective articles; operating gowns; mortuary pads; cable wrap; food tray pads; food preservation articles; seed germination pads; household pet litter; roofing materials; automotive trim; furniture; bedding; clothing; and soil modifiers.

Where the article of manufacture comprises a fibrous web made in accordance with the process of the above second aspect of the invention, one benefit that may be observed is that the binder is sufficient to retain the integrity

of the web during use but is sufficiently degradable in the presence of a large volume of water, particularly turbulent water such as is found in a toilet, that the article is particularly suitable for disposal in water, such as in a toilet.

The invention of the present invention will now be described with reference to the following examples.

**Examples 1 & 2**

A web of wood fluff containing a proportion of superabsorbent fibers of the type commercially available from Camelot Superabsorbent Limited was formed using a conventional airlaying machine. The web was then sprayed with water. The resultant web was tested for absorbency according to the dunk test and the Absorbency Capacity Index. In addition the wicking and tensile strength were measured. The composition of the webs and the results obtained are detailed in Table 1. In each case the sample was noted to be bound.

Table 1

| | Example 1 | Example 2 |
|---|---|---|
| GSM | 210 | 139 |
| Density | 0.08 | 0.14 |
| % Superabsorbent Fiber | 30 | 22.4 |
| ACI @ 3KPa (g/g) | 17.1 | |
| Dunk (g/g) | 26.3 | |
| Incline Distance (cm) @ 10 min | | 18.7 |
| Incline Weight (g) @ 10 min | | 28.1 |
| Tensile Peak Load (kgf) at MD | | 2.51 |
| Tensile Specific Strength ($kgf/cm^2$) @ MD | | 10.5 |
| Tensile Peak Load (kgf) at CD | | 3.01 |
| Tensile Specific Strength ($kgf/cm^2$) @ MD | | 12.5 |

The Absorbency Capacity Index (ACI) was measured as follows: The weight of the test pad was recorded (W0) and the pad was placed on a M/TS TEFO absorbency tester. A plate of weight equal to 0.1 kPa was placed on top of the test sample and 100 mls of 0.9% NaCl solution was run through the pipe in the center of the plate at a rate of 7 ml/sec. The "run-off" liquid was collected and weighed. After two minutes a further weight equal to 3 kPa was placed on the pad and the liquid collected after two minutes was recorded (W1). The volume of water W (3 kPa) = 100 - W1. The ACI at 3 kPa may be calculated as follows:

$$ACI\ (3\ kPa) = W\ (3\ kPa)\ /\ W$$

The Dunk test is carried out by immersing a pre-weighed sample in saline for 15 mins. The sample is then removed from the saline and allowed to "drip-dry" for 5 minutes. The sample is then reweighed and the value calculated.

The values of Incline distance and Incline weight represent the wicking properties of the sample. Wicking is measured by placing a 10cm x 30cm sample on a surface that is inclined at 30° to the horizontal. At the base of the slope 2cm of the sample are placed into a tray containing coloured saline. The dry weight of the sample is known. After 10 minutes the distance the saline has travelled along the sample and the weight of saline absorbed are noted.

The tensile strength of the sample are measured using standard ASTM techniques.

A comparison of the results obtained for the sample formed in accordance with the present invention with those obtained for webs formed by conventional processes indicates that the absorbency of the sample is not adversely effected by the use of water as a binding agent.

Whilst working with these samples it was noted that dust and other particles were not dispersed into the working environment to the extent that had been noted with samples produced in accordance with known methods.

## Claims

1. A process for anchoring water-absorbent material in a fibrous web, wherein the web comprises a preformed web including non water-absorbent fibers and water-absorbent material, wherein said water-absorbent material is superabsorbent, and said process comprises applying water to bind the water-absorbent material to the non water-absorbent fibers.

2. A process according to Claim 1 wherein the water-absorbent material is in fibrous form.

3. A process according to Claim 1 wherein water-absorbent material in particulate form is present within the web.

4. A process according to any one of Claims 1 to 3 wherein the water is applied to the web by immersing the web in water.

5. A process according to any one of Claims 1 to 3 wherein the water is applied to the web by spraying the web with water.

6. A process according to Claim 5 wherein the water is sprayed onto the fibrous web to obtain spot welds at the points where fibers are in mutual contact.

7. A process according to any one of Claims 1 to 3 wherein the water is applied to the web by passing the web through an area containing water-vapour.

8. A process according to any one of Claims 1 to 7 wherein one or more additional binder is used.

9. A process according to Claim 8 wherein the binder is applied to the web and cured before the water is applied.

10. A process according to Claim 8 wherein the water and the binder may be applied simultaneously.

11. A process according to any of the preceding claims wherein the fibrous web is water-absorbent paper.

12. A process according to any one of the preceding claims wherein the water-absorbent material is a copolymer containing from about 25 to 75 mole percent recurring units of an α-β unsaturated monomer and from about 75 to about 25 mole percent recurring units of a copolymerisable monomer.

13. A process according to Claim 12 wherein the α-β unsaturated monomers are those bearing at least one pendant carboxylic acid unit or derivative of a carboxylic acid unit.

14. A process according to Claim 13 wherein derivatives of carboxylic acid units include carboxylic acid salt groups, carboxylic acid amide groups, carboxylic acid imide groups, carboxylic acid anhydride groups and carboxylic acid ester groups.

15. A process according to any one of the preceding Claims wherein suitable α-β unsaturated monomers are selected from maleic acid, crotonic acid, fumaric acid, mesaconic acid, the sodium salt of maleic acid, the sodium salt of methyl 2-butene dicarboxylic acid, the sodium salt of itaconic acid, maleamic acid, malemide, N-phenyl maleimide, maleimide, maleic anhydride, fumaric anhydride, itaconic anhydride, citraconic anhydride, ethyl maleic anhydride, diethylmaleate, methylmaleate and mixtures thereof.

16. A process according to any one of the preceding Claims wherein the co-monomer is selected from ethylene, propylene, isobutylene, $C_1$ to $C_4$ alkyl methacrylates, vinyl acetates, methyl vinyl acetates, methyl vinyl ether, isobutyl vinyl ether and styrenic compounds having the formula:

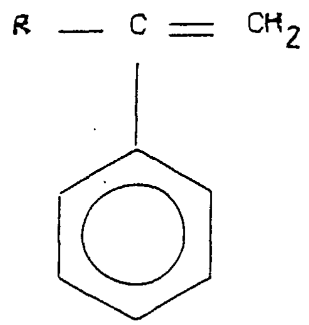

wherein R represents hydrogen or an alkyl group having 1 to 6 carbon atoms, and wherein the benzene ring is substituted or unsubstituted.

**17.** A process according to any one of the preceding claims wherein the copolymer is crosslinked with a cross-linking agent selected from monomers containing at least two hydroxyl groups such as alkylene glycols containing 2-10 carbon atoms and their ethers, cycloalkylene glycols, Bisphenol A, hydroxy alkylene derivatives of Bisphenol A, hydroquinone, phloroglucinol, hydroxy alkylene derivatives of diphenols, glycerol, erythritol, pentaerythritol, and mono-, di, or oligo-saccharides; heterocyclic carbonates; and monomers containing at least one amine group and at least one hydroxyl group such as ethanolamine, tris (hydroxymethyl) aminomethane, 3-amino-1-propanol, DL-1-amino-2-propanol, 2-amino-1-butanol, N, N-dimethylethanolamine,diisopropanol-amine methyl diethanol amine, triethanol amine and 2-(methylamino)ethanol.

**18.** An article of manufacture comprising a fibrous web made in accordance with the process of any one of Claims 1 to 17.

**19.** An article according to Claim 18 wherein the article of manufacture is selected from the group consisting of: disposable diapers; sanitary napkins; tampons; pant liners; adult incontinence pads; coverstock for feminine hygiene products; surgical and dental sponges; bandages; patient underpads; wipes; domestic wipes; industrial wipes; packaging; medical tray pads; fenestration drapes; filters; spill control materials; waste management materials; protective articles; operating gowns; mortuary pads; cable wrap; food tray pads; food preservation articles; seed germination pads; household pet litter; roofing materials; automotive trim; furniture; bedding; clothing; and soil modifiers.

**Patentansprüche**

**1.** Verfahren zur Verankerung von wasserabsorbierendem Material in einer Faserstoffbahn, wobei die Bahn eine vorgefertigte Bahn mit nicht wasserabsorbierenden Fasern und wasserabsorbierendem Material umfaßt, wobei das wasserabsorbierende Material superabsorbierend ist und das Verfahren die Anwendung von Wasser umfaßt, um das wasserabsorbierende Material an die nicht wasserabsorbierenden Fasern zu binden.

**2.** Verfahren nach Anspruch 1, bei dem das wasserabsorbierende Material faserförmig ist.

**3.** Verfahren nach Anspruch 1, bei dem das wasserabsorbierende Material in der Bahn in Teilchenform vorliegt.

**4.** Verfahren nach einem der Ansprüche 1 bis 3, bei dem das Wasser durch Eintauchen der Bahn in Wasser auf die Bahn aufgebracht wird.

**5.** Verfahren nach einem der Ansprüche 1 bis 3, bei dem das Wasser durch Besprühen der Bahn mit Wasser auf die Bahn aufgebracht wird.

**6.** Verfahren nach Anspruch 5, bei dem das Wasser auf die Faserstoffbahn gesprüht wird, um Verschweißungspunkte an den Stellen zu erhalten, wo die Fasern in gegenseitigem Kontakt sind.

**7.** Verfahren nach einem der Ansprüche 1 bis 3, bei dem das Wasser auf die Bahn aufgebracht wird, indem die Bahn

einen Wasserdampf enthaltenden Bereich passiert.

**8.** Verfahren nach einem der Ansprüche 1 bis 7, bei dem ein oder mehrere zusätzliche(r) Binder verwendet werden.

**9.** Verfahren nach Anspruch 8, bei dem der Binder auf die Bahn aufgebracht wird und aushärtet, bevor das Wasser aufgebracht wird.

**10.** Verfahren nach Anspruch 8, bei dem das Wasser und der Binder gleichzeitig aufgebracht werden können.

**11.** Verfahren nach einem der vorstehenden Ansprüche, bei dem die Faserstoffbahn wasserabsorbierendes Papier ist.

**12.** Verfahren nach einem der vorstehenden Ansprüche, bei dem das wasserabsorbierende Material ein Copolymer mit etwa 25 bis 75 Mol-% Wiederholungseinheiten eines $\alpha,\beta$-ungesättigten Monomers und etwa 75 bis etwa 25 Mol-% Wiederholungseinheiten eines copolymerisierbaren Monomers ist.

**13.** Verfahren nach Anspruch 12, bei dem die $\alpha,\beta$-ungesättigten Monomere solche sind, die mindestens eine seitenständige Carbonsäureeinheit oder ein Derivat einer Carbonsäureeinheit tragen

**14.** Verfahren nach Anspruch 13, bei dem Derivate der Carbonsäureeinheiten Carbonsäuresalzgruppen, Carbonsäureamidgruppen, Carbonsäureimidgruppen, Carbonsäureanhydridgruppen und Carbonsäureestergruppen umfassen.

**15.** Verfahren nach einem der vorstehenden Ansprüche, bei dem geeignete $\alpha,\beta$-ungesättigte Monomere ausgewählt werden unter Maleinsäure, Crotonsäure, Fumarsäure, Mesaconsäure, dem Natriumsalz der Maleinsäure, dem Natriumsalz der Methyl-2-butendicarbonsäure, dem Natriumsalz der Itaconsäure, Maleinsäureamid, Malemid, N-Phenylmaleimid, Maleimid, Maleinsäureanhydrid, Fumarsäureanhydrid, Itaconsäureanhydrid, Citraconsäureanhydrid, Ethylmaleinsäureanhydrid, Diethylmaleat, Methylmaleat und Gemischen davon.

**16.** Verfahren nach einem der vorstehenden Ansprüche, bei dem das Comonomer ausgewählt wird unter Ethylen, Propylen, Isobutylen, $C_1$- bis $C_4$-Alkylmethacrylaten, Vinylacetaten, Methylvinylacetaten, Methylvinylether, Isobutylvinylether und Styrolverbindungen der Formel:

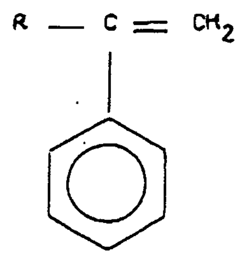

in der R Wasserstoff oder eine Alkylgruppe mit 1 bis 6 Kohlenstoffatomen ist, und in der der Benzolring substituiert oder nicht substituiert ist.

**17.** Verfahren nach einem der vorstehenden Ansprüche, bei dem das Copolymer vernetzt wird mit einem Vernetzungsmittel, das ausgewählt wird unter den Monomeren mit mindestens zwei Hydroxylgruppen wie Alkylenglykolen mit 2 bis 10 Kohlenstoffatomen und deren Ethern, Cycloalkylenglykolen, Bisphenol A, Hydroxyalkylenderivaten von Bisphenol A, Hydrochinon, Phloroglucinol, Hydroxyalkylenderivaten von Diphenolen, Glycerin, Erythritol, Pentaerythritol, und Mono-, Di-, oder Oligosacchariden; heterocyclischen Carbonaten; und Monomeren mit mindestens einer Amingruppe und mindestens einer Hydroxylgruppe wie Ethanolamin, Tris (hydroxymethyl) -aminomethan, 3-Amino-1-propanol, DL-1-Amino-2-propanol, 2-Amino-1-butanol, N,N-Dimethylethanolamin, Diisopronanolamin, Methyldiethanolamin, Triethanolamin und 2- (Methylamino)ethanol.

**18.** Herstellungsgegenstand, umfassend eine Faserstoffbahn, die gemäß dem Verfahren nach einem der Ansprüche 1 bis 17 hergestellt wird.

19. Gegenstand nach Anspruch 18, wobei der Herstellungsgegenstand ausgewählt wird aus der Gruppe bestehend aus: Wegwerfwindeln; Monatsbinden; Tampons; Slipeinlagen; Inkontinenzunterlagen für Erwachsene; Abdeckmaterial für Hygieneprodukte für Frauen; chirurgische und zahnärztliche Schwämme; Bandagen; Unterlagen für Patienten; Wischtücher; Wischtücher für den Haushalt; industrielle Wischtücher; Verpackungsmaterial; Unterlagen für medizinische Tabletts; Fenstervorhangstoffe; Filter; Materialien für die Überlaufkontrolle; Materialien für die Abfallhandhabung; Schutzartikel; Operationskleidung; Unterlagen für Leichenhäuser; Kabelumhüllungen; Unterlagen für Esstabletts; Artikel für die Konservierung von Lebensmitteln, Unterlagen für die Keimung von Samen; Haustierstreu; Dach- bzw. Abdeckmaterialien; Kraftfahrzeug-Ausstattungen; Mobiliar; Bettzeug; Kleidung; und Bodenmodifizierer.

**Revendications**

1. Procédé pour l'ancrage ou la fixation d'un matériau absorbant l'eau à l'intérieur d'une bande fibreuse, dans lequel la bande comprend une bande préformée incorporant des fibres n'absorbant pas l'eau et un matériau absorbant l'eau, dans lequel ledit matériau absorbant l'eau est supra-absorbant et ledit procédé comprend l'application de l'eau pour lier le matériau absorbant l'eau aux fibres n'absorbant pas l'eau.

2. Procédé selon la revendication 1, dans lequel le matériau absorbant l'eau est sous la forme fibreuse.

3. Procédé selon la revendication 1, dans lequel un matériau absorbant l'eau est présent sous la forme particulaire à l'intérieur de la bande.

4. Procédé selon l'une quelconque des revendications 1 à 3, dans lequel l'eau est appliquée sur la bande par immersion de cette dernière dans l'eau.

5. Procédé selon l'une quelconque des revendications 1 à 3, dans lequel l'eau est appliquée sur la bande par pulvérisation d'eau sur celle-ci.

6. Procédé selon la revendication 5, dans lequel l'eau est pulvérisée sur la bande fibreuse pour obtenir des soudures par points aux endroits où les fibres sont en contact entre elles.

7. Procédé selon l'une quelconque des revendications 1 à 3, dans lequel l'eau est appliquée sur la bande en faisant passer celle-ci à travers une zone contenant de la vapeur d'eau.

8. Procédé selon l'une quelconque des revendications 1 à 7, dans lequel on utilise un ou plusieurs liants supplémentaires.

9. Procédé selon la revendication 8, dans lequel le liant est appliqué sur la bande et il séché avant l'application de l'eau.

10. Procédé selon la revendication 8, dans lequel l'eau et le liant peuvent être appliqués simultanément.

11. Procédé selon l'une quelconque des revendications précédentes, dans lequel la bande fibreuse est du papier absorbant l'eau.

12. Procédé selon l'une quelconque des revendications précédentes, dans lequel le matériau absorbant l'eau est un copolymère contenant d''environ 25 à 75 pour cent en mole de motifs récurrents d'un monomère insaturé $\alpha$ - $\beta$ et d'environ 75 à environ 25 pour cent en mole de motifs récurrents d'un monomère copolymérisable.

13. Procédé selon la revendication 12, dans lequel les monomères insaturés $\alpha$-$\beta$ sont ceux portant au moins une unité d'acide carboxylique présente ou un dérivé d'une unité d'acide carboxylique.

14. Procédé selon la revendication 13, dans lequel les dérivés des unités d'acide carboxylique comprennent des groupes de sel de l'acide carboxylique, des groupes amide de l'acide carboxylique, des groupes imide de l'acide carboxylique, des groupes anhydride de l'acide carboxylique et des groupes ester de l'acide carboxylique.

15. Procédé selon l'une quelconque des revendications précédentes, dans lequel des monomères insaturés $\alpha$ - $\beta$ sont

choisis à partir de l'acide maléique, de l'acide crotonique, de l'acide fumarique, de l'acide mésaconique, du sel de sodium de l'acide maléique, du sel de sodium de l'acide méthy12-butène dicarboxylique, du sel de sodium de l'acide itaconique, de l'acide maléamique, du malémide, du N-phényl maléimide, du maléimide, de l'acide maléique anhydride, de l'acide fumarique anhydride, de l'acide itaconique anhydride, de l'acide citraconique anhydride, de l'acide éthyl maléique anhydride, du diéthylmaléate, du méthylmaléate et de leurs mélanges.

**16.** Procédé selon l'une quelconque des revendications précédentes, dans lequel le comonomère est choisi à partir d'éthylène, de propylène, d'isobutylène, d'alkylméthacrylates en $C_1$ à $C_4$, de vinylacétates, de méthylvinylacétates, de méthylvinyléther, d'isobutylvinyléther et des composés styréniques ayant la formule :

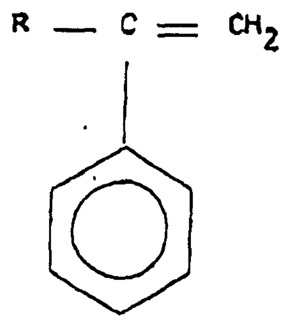

Dans laquelle R représente l'hydrogène ou un groupe alkyle ayant 1 à 6 atomes de carbone et dans laquelle le cycle benzène est substitué ou non substitué.

**17.** Procédé selon l'une quelconque des revendications précédentes, dans lequel le copolymère est réticulé au moyen d'un agent de réticulation choisi à partir de monomères contenant au moins deux groupes hydroxyle tels que des alkylène - glycols contenant 2 - 10 atomes de carbone et leurs éthers, des cycloalkylènes - glycols, du Bisphénol A, des dérivés hydroxy alkylènes de Bisphénol A, de l'hydroquinone, du phloroglucinol, des dérivés hydroxy alkylènes de diphénols, du glycérol, de l'érythritol, du pentaérythritol, et des mono-,di-, ou oligosaccharides; des carbonates hétérocycliques; et des monomères contenant au moins un groupe amino et au moins un groupe hydroxyle tel que l'éthanolamine, le tris (hydroxyméthyl) aminométhane, le 3-amino-1-propanol, le DL-1-amino-2-propanol, le 2-amino-1-butanol, la N,N-diméthyléthanolamine, la diisopropanolamine, la méthyldiéthanolamine, la triéthanolamine et le 2-(méthylamino)éthanol.

**18.** Un article de fabrication comprenant une bande fibreuse réalisée selon le procédé de l'une quelconque des revendications 1 à 17.

**19.** Article selon la revendication 18, dans lequel l'article de fabrication est choisi à partir du groupe constitué par : les couches jetables; les serviettes hygiéniques; les tampons; les garnitures de culottes; les garnitures d'incontinence pour adultes; les recouvrements protecteurs pour produits hygiéniques féminins; les éponges chirurgicales et dentaires; bandages ou pansements; les garnitures pour patients; les torchons ou essuie-tout; les torchons ou essuie-tout domestiques; les torchons ou essuie-tout industriels; les emballages; les protège - plateaux médicaux; les rideaux ou tentures pour fenêtrage; les filtres; les matériaux de contrôle de trop-plein; les matériaux pour la gestion des déchets; les articles de protection; les vêtements de salle opératoire; les coussins mortuaires; les enrobages de câbles; les protège - plateaux de nourriture; les articles pour la conservation de produits alimentaires; les supports de germination de graines ou semences; les litières pour animaux domestiques; les matériaux pour toitures; les garnitures pour automobiles; les meubles; la literie; les vêtements; et les agents de modification des sols.